# EUROPEAN PATENT SPECIFICATION

(11) **EP 2 420 190 B1**
(45) Date of publication and mention of the grant of the patent: **20.03.2019**
(21) Application number: 11177139.0
(22) Date of filing: 10.08.2011
(51) Int. Cl.: A61B 10/00, A61B 5/00, B01L 3/00, G01N 33/50, G01N 35/02

(54) **Physiologically active substance collecting device**
Physiologisch aktive Substanzsammelvorrichtung
Dispositif de collecte de substance active physiologique

(30) Priority: 18.08.2010 JP 2010183077
(43) Date of publication of application: 22.02.2012
(73) Proprietor: Sony Corporation, Tokyo 108-0075 (JP)
(72) Inventor: Matsumoto, Masahiro, Tokyo (JP); Katsuhara, Tomoko, Tokyo (JP); Watanabe, Yuuki, Tokyo (JP)
(74) Representative: D Young & Co LLP

(56) References cited:
- EP-A1- 2 124 053
- WO-A1-96/32147
- WO-A1-2009/076469
- US-A- 5 998 217
- US-A- 6 133 045
- US-A1- 2004 069 076

## Description

### FIELD

The present disclosure relates to physiologically active substance collecting devices, specifically to physiologically active substance collecting devices used to acquire physiologically active substances from the body surface of a living organism.

### BACKGROUND

Known methods of acquiring information concerning stress, emotion, menstrual cycle, and other conditions of a living organism (hereinafter, "information concerning a living organism" or, simply, "biological information") include biological information acquisition methods that are based on psychological evaluations involving, for example, questioning and sensory questionnaires, physiological tests measuring, for example, brain waves or myoelectricity, and behavior measurements involving the use of, for example, a work record. For example, JP-A-2006-94969 (Patent Document 1) discloses a technique that determines the menstrual cycle based on heart rates. Japanese Patent No. 2582957 (Patent Document 2) discloses a life activity monitoring system that monitors body temperature fluctuations and heart rates.

In recent years, simpler techniques have been developed that acquire information concerning a living organism with the use of a physiologically active substance contained in blood, urine, or saliva as an index. For example, JP-A-11-38004 (Patent Document 3) discloses a method for quantifying stress using the concentration of adrenal cortical steroid and/or its metabolic products in saliva as an index. JP-A-2000-131318 (Patent Document 4) discloses a method that allows the stress level to be grasped as either "comfortable" or "uncomfortable" using biological substances such as β-endorphin, dopamine, immunoglobulin A, and prostaglandin D2 contained in blood or in other body parts as an index.

The biological information acquisition methods in which the physiologically active substances contained in blood, urine, or saliva are used as an index are advantageous, because these methods are simpler than methods involving psychological evaluations, physiological tests, or behavior measurements, and do not require large devices.

On the other hand, the methods require the procedure of collecting blood, urine, and saliva for the quantification of physiologically active substances. For example, when blood is used, blood collection can be mentally or physically demanding to a subject. The mental and physical load associated with blood collection may itself be perceived as stress, and may cause changes in the subject's conditions, including stress and emotion, and prevent accurate acquisition of biological information.

Use of urine and saliva can circumvent the problematic medical practice issue raised in blood collection, and can reduce the mental and physical load put on the subject. It is, however, difficult to collect urine and saliva over a time course or on a steady basis, and, because there is a time lag between the collection of urine or saliva and the body's metabolism of the physiologically active substance contained in urine or saliva, it is difficult to acquire biological information in real-time. Further, even though urine or saliva collection does not produce as much mental or physical load as blood collection, it still makes the subject strongly aware of the collection procedure, presenting difficulties in the accurate acquisition of biological information.
EP 2 124 053 A1 discloses a biological information acquisition method and instrument for acquiring information on a living body on a basis of a quantified value of a physiologically active substance originated from the living body, includes a step of collecting the physiologically active substance from a body surface of the living body.
WO 2009/076469 A1 discloses a tissue, such as an amniotic or chorionic membrane, harvesting device that integrates a system facilitating subsequent storage of tissue samples. The device cuts a sample of the target tissue and automatically deposits the sample in a storage vessel.

### SUMMARY OF THE INVENTION

Aspects of the invention set out in the appended claims.

Embodiments of the present disclosure provide a physiologically active substance collecting device that can be used to collect physiologically active substances from a living organism on a steady basis in a convenient and minimally invasive manner.

According to an embodiment of the present disclosure, there is provided a physiologically active substance collecting device that includes : a collecting section brought into contact with a body surface of a living organism to acquire a physiologically active substance from the body surface; and liquid sending means for sending a solvent to the collecting section, the collecting section having an aperture at which the solvent flown by being sent from the liquid sending means contacts the body surface. In the physiologically active substance collecting device, the solvent is contacted to the body surface of a living organism at the collecting section to enable the collection of the physiologically active substance into the solvent.

In one embodiment the physiologically active substance collecting device further includes a collecting unit with which the solvent contacted the body surface at the aperture is injected into a container sealed by an elastic, self-sealing sealant that undergoes elastic deformation, wherein the collecting unit includes a hollow needle capable of penetrating through the sealant, and drains the solvent through the hole of the hollow needle at a needle end.

In one embodiment the physiologically active substance collecting device further includes: a first driving unit that moves the hollow needle downward to place the needle end inside the container through the sealant, and upward to pull back the needle end out of the container through the sealant; and a feeder that places the container one by one underneath the hollow needle.

In one embodiment the physiologically active substance collecting device further includes: a waste liquid receptor positioned between the hollow needle and the container when the needle end of the hollow needle is out of the container, so as to receive the solvent drained through the hole; and a second driving unit that retreats the waste liquid receptor from between the hollow needle and the container in advance of the hollow needle moving downward.

The physiologically active substance collecting device further includes an air sending unit that sends air to the collecting section to selectively introduce the solvent and air into the collecting section. By selectively introducing the solvent and air into the collecting section, the solvent that comes into contact with the body surface of a living organism at the collecting section can be collected in a flow separated by air in predetermined volumes .

In one embodiment, in the physiologically active substance collecting device, the collecting section includes a substrate that includes a channel that flows the solvent, a solvent inlet for the channel, a solvent outlet for the channel, and the aperture positioned between the inlet and the outlet of the channel, and that the substrate is replaceable from the collecting section.

The physiologically active substance collecting device may further include: a quantifying section that quantifies the physiologically active substance; and a determining section that automatically determines and acquires information concerning the living organism based on the quantified value of the physiologically active substance.

Here, the physiologically active substance may be, for example, cortisol, monoamine, estrogen, or growth hormone. In this way, the information concerning the stress, emotion, menstrual cycle, exercise effect in the living organism can be acquired as the information.

As used herein, "information concerning a living organism" encompasses not only information concerning, for example, stress, emotion, menstrual cycle, and exercise effect, but information concerning sleepiness (wakefulness level), health condition, and circadian rhythm (biological rhythm). The meaning of "emotion" encompasses, for example, excitement, fear, anger, aggression, comfort, and anxiety.

The "physiologically active substance" includes a wide range of substances present in a living organism, and that have physiological effects and pharmacological effects on the living organism to take part in changes in the state of a living organism, including stress, emotion, menstrual cycle, and metabolism. Specific examples of the physiologically active substance include steroid hormones such as cortisol and estradiol, catecholamines such as adrenaline and dopamine, and physiologically active peptides such as oxytocin and endorphin (see Table 1 below).

The physiologically active substance collecting device according to the embodiment of the present disclosure can thus be used to collect physiologically active substances from a living organism on a steady basis in a convenient and minimally invasive manner.

Further particular and preferred aspects of the present invention are set out in the accompanying independent and dependent claims. Features of the dependent claims may be combined with features of the independent claims as appropriate, and in combinations other than those explicitly set out in the claims.

### BRIEF DESCRIPTION OF THE DRAWINGS

The present invention will be described further, by way of example only, with reference to preferred embodiments thereof as illustrated in the accompanying drawings, in which:
FIG. 1 is a perspective view explaining the schematic structure of a physiologically active substance collecting device according to First Embodiment of the present disclosure.
FIG. 2 is a block diagram explaining a flow of a solvent in the physiologically active substance collecting device.
FIGS. 3A and 3B are schematic views explaining the configuration of a collecting section.
FIG. 4 is a schematic view explaining the procedure of acquiring a physiologically active substance from a body surface.
FIGS. 5A and 5B are schematic views explaining the configuration of a holder; FIG. 5C is a schematic view explaining the operation of the holder.
FIGS. 6A and 6B are schematic views explaining the configuration of a collecting unit that includes a hollow needle.
FIG. 7 is a schematic view explaining the operation of the collecting unit that includes a hollow needle.
FIG. 8 is a schematic view explaining the configuration of a variation of the collecting section.
FIGS. 9A and 9B are schematic views explaining the configuration of another variation of the collecting section.
FIGS. 10A and 10B are schematic views explaining the configuration of yet another variation of the collecting section.
FIGS. 11A and 11B are schematic views explaining methods of acquiring a physiologically active substance from the skin surface of a finger (Example 1).
FIG. 12 is a diagram representing the results of the measurement of cortisol level in one subject using high-performance liquid chromatography (HPLC) (Example 1).
FIG. 13 in A to F represents the results of the measurement of cortisol level in six subjects using high-performance liquid chromatography (HPLC) (Example 1).
FIG. 14 is a diagram representing SPR curves obtained from a standard cortisol solution (Example 1).
FIGS. 15A and 15B are diagrams representing a plot of SPR shifts, and a standard curve obtained from a standard cortisol solution (Example 1).
FIG. 16 is a diagram representing the results of the measurement of norepinephrine level and L-DOPA level using high-performance liquid chromatography (HPLC) (Example 2).
FIG. 17 is a diagram representing the results of the measurement of serotonin level using high-performance liquid chromatography (HPLC) (Example 3).
FIG. 18 is a diagram representing the results of the measurement of estradiol level using enzyme immunoassay (ELISA) (Example 4).
FIG. 19 is a diagram representing the results of the measurement of growth hormone level using enzyme immunoassay (ELISA) (Example 5).

### DETAILED DESCRIPTION

The following will describe a preferred embodiment of the present disclosure with reference to the accompanying drawings. It should be noted that the embodiment below is merely an illustrative representation of the present disclosure, and should not be construed to narrow the scope of the present disclosure. Descriptions will be given in the following order.
1. Physiologically Active Substance Collecting Device According to First Embodiment of the Present Disclosure
   (1) Overview
   (2) Overall configuration
   (3) Collecting section
   (4) Collecting unit
   (5) Quantifying section and determining section
2. Physiologically Active Substance Collecting Device According to Variations of First Embodiment
   (1) First Variation
   (2) Second Variation
   (3) Third Variation
3. Physiologically Active Substance and Biological Information

### 1. Physiologically Active Substance Collecting Device According to First Embodiment of the Present Disclosure

### (1) Overview

For the accurate sensing of biological information, the present inventors conducted intensive studies on techniques for collecting physiologically active substances from a living organism. The inventors found, for the first time, that physiologically active substances could be acquired from body surfaces such as finger and palm surfaces, as will be described in detail later in Examples.

It is common practice to acquire physiologically active substances from bodily fluids such as blood, urine, and saliva. To the knowledge of the present inventors, there is no report of acquiring physiologically active substances from the body surface of a living organism.

The detailed mechanism by which physiologically active substances are acquired from the body surface remains elusive. However, there is a possibility that the physiologically active substance secreted into, for example, sweat and sebum may be present on the body surface. There is another possibility that the physiologically active substance in blood passes through body surface cells to reach the body surface. Because many of the physiologically active substances are soluble in lipid and permeable through cell membrane, it is highly probable that the physiologically active substance acquired from the body surface is one that is secreted into sebum or that has passed through the cells.

The present disclosure has been made based on these new findings, by recognizing the need for a physiologically active substance collecting device that can be used to acquire physiologically active substances from the body surface of a living organism.

### (2) Overall Configuration

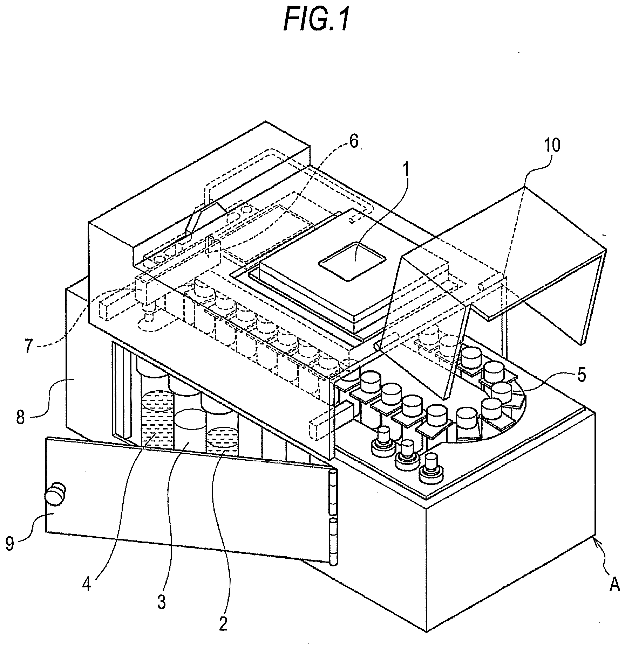

FIG. 1 is a perspective view explaining the schematic structure of a physiologically active substance collecting device according to First Embodiment of the present disclosure.

In FIG. 1, a physiologically active substance collecting device A is configured to include a collecting section 1 brought into contact with the surface of a living organism (hereinafter, also referred to as "body surface") to acquire a physiologically active substance from the body surface, a liquid sending unit that sends a solvent to the collecting section 1, an air sending unit that sends air to the collecting section 1, and a collecting unit with which the solvent that has contacted the body surface at the collecting section 1 is injected into containers 5. The solvent may be water or various organic solvents. For example, ethanol water can be used.

In FIG. 1, a solvent tank 2 is provided as a component of the liquid sending unit. In addition to the solvent tank 2, the liquid sending unit is also configured from other components such as pumps, tubes, and valves used to send the solvent inside the solvent tank 2 to the collecting section 1. An air tank 3 is provided as a component of the air sending unit. In addition to the air tank 3, the air sending unit is also configured from other components such as pumps, tubes, and valves used to send air inside the air tank 3 to the collecting section 1. The air tank 3 serves as a filter that prevents dust and foreign particles from being sucked into the tubes or valves.

In FIG. 1, a hollow needle 6 is provided as a component of the collecting unit. In addition to the hollow needle 6, the collecting unit is also configured from other components such as pumps, tubes, and valves used to send the solvent contacted to the body surface at the collecting section 1 to the hollow needle 6. The hollow needle 6 is configured so that the solvent sent from the collecting section 1 after contacted the body surface is drained through the hole at the needle end, and serves to inject the solvent into the containers 5 disposed underneath.

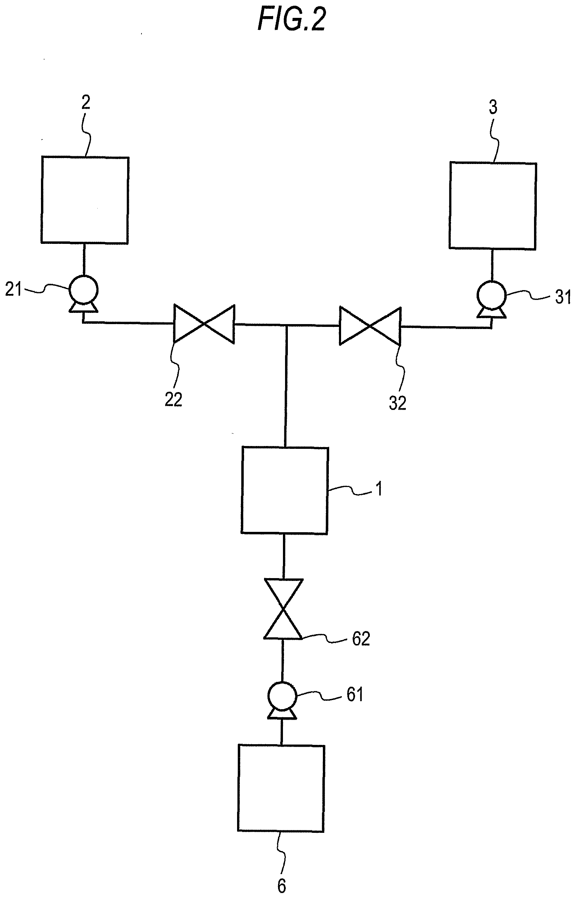

FIG. 2 is a block diagram representing a flow of the solvent in the physiologically active substance collecting device A.

The collecting section 1 is connected to a channel used to send liquid from the solvent tank 2. The collecting section 1 is also connected to a channel used to send air from the air tank 3. The collecting section 1 is also connected to a channel through which the solvent contacted the body surface, and air are sent to the hollow needle 6. General-purpose tubes can be used for these channels. A general-purpose pump (pumps 21, 31, 61) is provided for each channel to sent liquid or air.

The tube connecting the solvent tank 2 to the collecting section 1, and the tube connecting the air tank 3 to the collecting section 1 merge on the upstream side of the collecting section 1. Referring to the figure, valves 22 and 32 are provided on the way to the junction from the solvent tank 2 and the air tank 3. In the physiologically active substance collecting device A, the valves 22 and 32 are opened and closed under the control of a system control unit(not illustrated) to selectively introduce the solvent and air to the collecting section 1. In the figure, a valve 62 is provided for the tube that connects the collecting section 1 to the hollow needle 6. The valve 62 is opened and closed under the control of the system control unit, and serves to start and stop the draining of the solvent from the hollow needle 6. The system control unit is provided inside a control box 8 illustrated in FIG. 1.

In the physiologically active substance collecting device A, it is preferable that the tubes, valves, and pumps through which the solvent is flown be made of material that does not easily attract the physiologically active substance, or be subjected to a surface treatment that makes the adsorption of the physiologically active substance difficult.

Referring back to FIG. 1, the hollow needle 6 is movable along the vertical direction. The hollow needle 6 moves downward when the solvent sent from the collecting section 1 after contacting the body surface is injected into the container 5. With the hollow needle 6 down, the needle end is inserted in the container 5 disposed underneath, and the solvent drained through the hole at the needle end is introduced into the container 5.

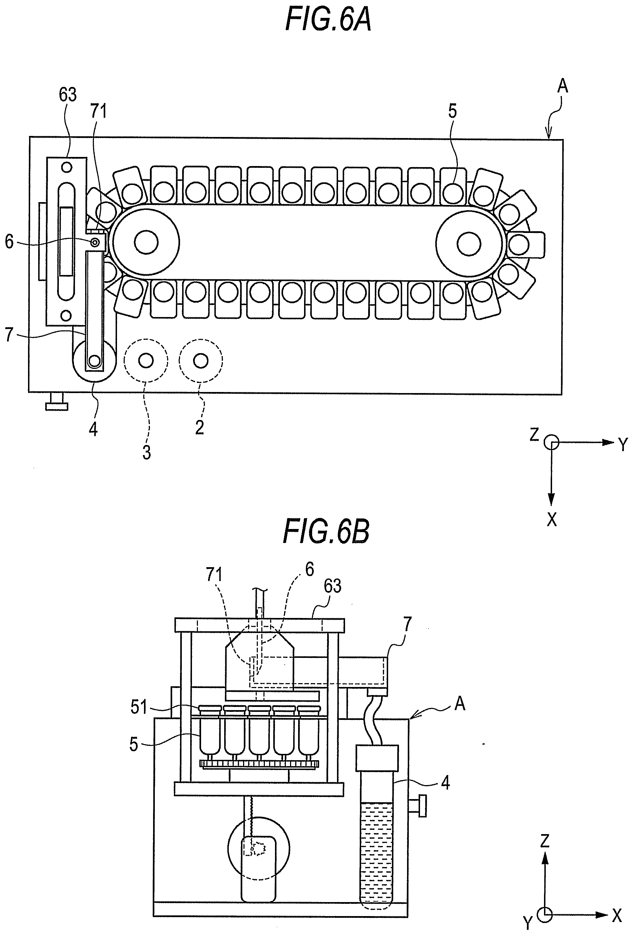

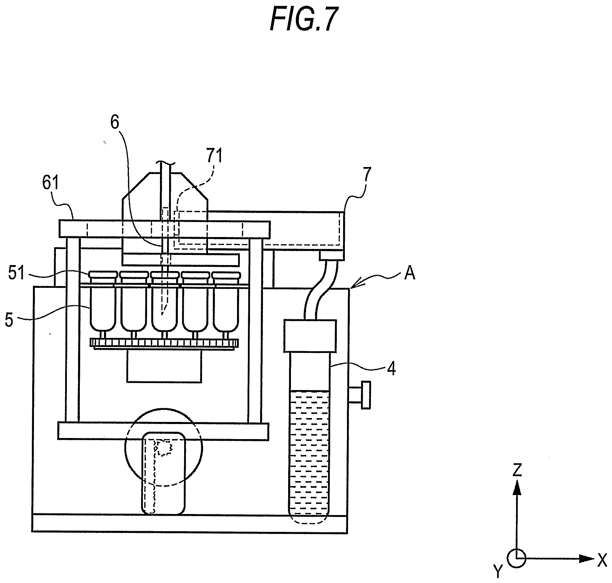

On the other hand, with the hollow needle 6 up and the needle end out of the container 5, the solvent drained through the hole at the needle end is received by a waste liquid receptor 7 (hereinafter, "waste liquid tray 7") positioned between the hollow needle 6 and the container 5 disposed underneath. The solvent collected by the waste liquid tray 7 is sent to a waste liquid tank 4 and stored therein. The waste liquid tray 7 is configured to retreat from between the hollow needle 6 and the container 5 prior to the downward movement of the hollow needle 6, in order not to interfere with the vertical movement of the hollow needle 6 (will be described in detail with reference to FIGS. 6A and 6B and FIG. 7) .

Preferably, more than one container 5 is installed in the physiologically active substance collecting device A. By installing more than one container 5, the physiologically active substance collecting device A can collect more sample volume, or can collect samples from different living organisms or samples from different sites of the body surface in each different container. With more than one container 5, it is also possible to collect samples from the same living organism or body surface with the passage of time or at different time points. Note that, as used herein, the "sample" means a solvent that has contacted the body surface and contains a biological substance, and encompasses solvents collected for comparison without being contacted with the body surface.

In the present embodiment, a conveyer chain (feeder) is provided that sets the container 5 in position underneath the hollow needle 6, one by one. As the conveyer chain successively feeds the container 5 underneath the hollow needle 6, the hollow needle 6 is moved up and down to successively inject the sample in each container 5 (will be described later in more detail with reference to FIGS. 6A and 6B) . Note that the feeder is not limited to the conveyer chain, and can be realized by a wide range of means, as long as the container 5 can be fed one by one underneath the hollow needle 6.

In the figure, an upper cover 10 covers the upper part of the physiologically active substance collecting device A. The upper cover 10 can be opened and closed to install the containers 5 on the conveyer chain, and to remove the containers 5 from the conveyer chain. A lower cover 9 is opened and closed to replace the solvent tank 2, the air tank 3, and the waste liquid tank 4.

### (3) Collecting section

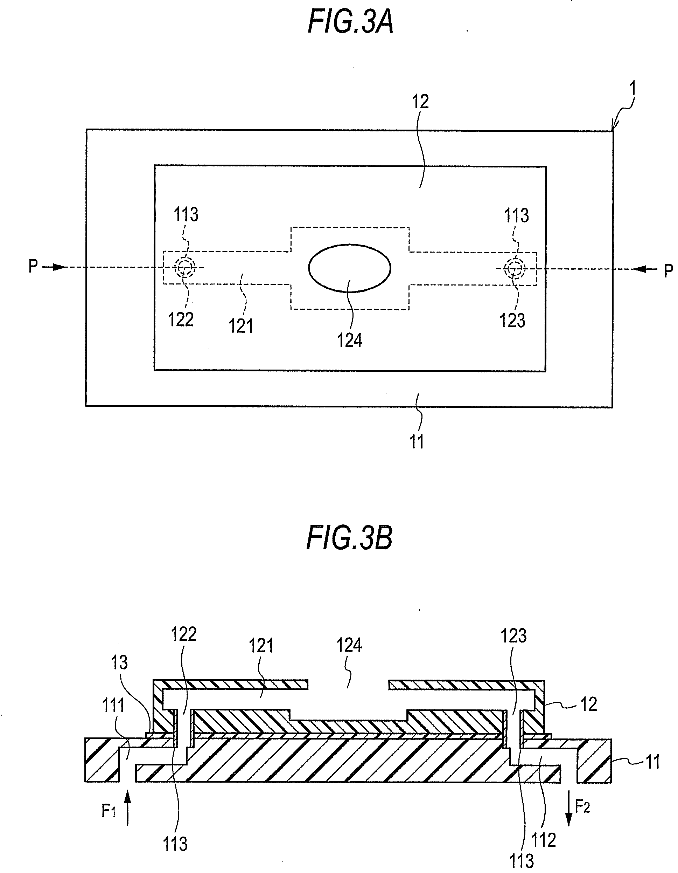

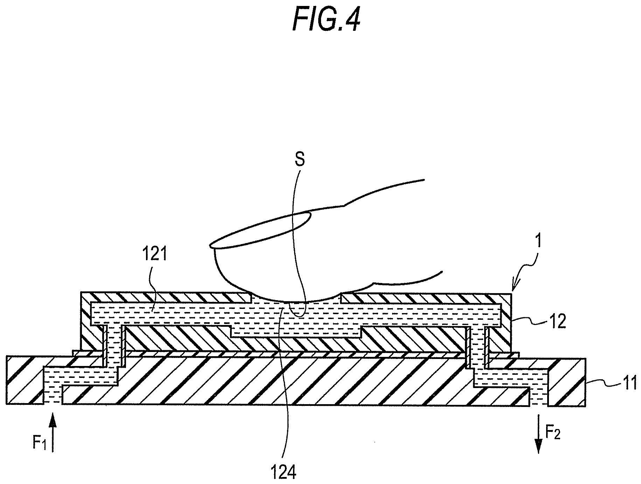

The configuration of the collecting section 1 is described below with reference to FIGS. 3A and 3B, and FIG. 4. FIGS. 3A and 3B are schematic views explaining the configuration of the collecting section 1, in which FIG. 3A shows a top view, and FIG. 3B shows a cross sectional view taken at P-P in FIG. 3A. FIG. 4 is a cross sectional schematic view explaining the procedure of acquiring the physiologically active substance from the body surface.

The two main components of the collecting section 1 are an anchor substrate 11 and a collection substrate 12. The anchor substrate 11 is provided by being anchored to the main body of the physiologically active substance collecting device A. The collection substrate 12 is detachably provided on the anchor substrate 11, and is replaceable.

The collection substrate 12 includes a channel 121 that flows the solvent sent thereto, an inlet 122 for the solvent flowing into the channel 121, and an outlet 123 for the solvent flowing out of the channel 121. The anchor substrate 11 includes a channel 111 through which the solvent sent from the solvent tank 2 is flown, and a channel 112 through which the solvent sent to the hollow needle 6 is flown. In the figures, arrows F₁ and F₂ indicate the flow directions of the solvent sent to or drained from the channels in the anchor substrate 11.

The collection substrate 12 has an aperture 124 provided between the inlet 122 and the outlet 123 of the channel 121, and that opens to outside on the upper side of the substrate. The aperture 124 serves to bring the solvent flown in the channel 121 into contact with the body surface. Specifically, as illustrated in FIG. 4, with the body surface S tightly attached to the aperture 124 of the channel 121 flowing the solvent, the solvent filling the channel 121 contacts the body surface S, and the physiologically active substance present on the body surface S is collected in the solvent. The solvent brought into contact with the body surface S is then sent to the hollow needle 6, as indicated by arrow F₂.

Here, by selectively introducing the solvent and air from the solvent tank 2 and the air tank 3 into the collecting section 1 using the valves 22 and 32 opened and closed under control as described with reference to FIG. 2, the solvent in contact with the body surface S can be sent to the hollow needle 6 by being separated into predetermined volumes by air. Specifically, with the body surface S tightly attached to the aperture 124, a predetermined volume of solvent is sent to the channel 121. Air is then sent into the channel 121 to separate the flow of the solvent in the channel 121 by air. A predetermined volume of solvent is then resent into the channel 121. By introducing air and the solvent in turn, the solvent that has contacted the body surface S can be sent out to the hollow needle 6 in predetermined volumes separated by air. In this way, the physiologically active substance in the collected sample can be suppressed from being diluted, and samples collected from different living organisms or from different sites of the body surface can be divided and collected in different containers.

The body surface S has been described as being a finger tip. However, the body surface S as the acquisition site of the physiologically active substance is not particularly limited, though skin surface such as finger and palm surface is convenient. The aperture 124 preferably has a shape that allows the skin surface at the acquisition site to be tightly attached, depending on the acquisition site of the physiologically active substance. For more tight attachment to the aperture 124, the body surface S may be fixed to the collection substrate 12 using, for example, an adhesive tape or a band.

As described above, the collection substrate 12 is disposed on the anchor substrate 11, and is replaceable. In this way, different collection substrates 12 with different shapes of apertures 124 can be appropriately replaced and attached according to, for example, the shape and size of the acquisition site of the physiologically active substance. Further, the collection substrate 12 can be replaced to a new one for each sample collection, and cross contamination between the samples can be prevented when collecting samples from different living organisms or from different sites of the body surface, or when collecting samples from the same living organism or body surface at different times. Note that when the collection substrate 12 is not replaced, it is desirable that washing be performed by flowing the solvent or a washing liquid through the collecting section 1 for a predetermined time period, in order to prevent cross contamination between samples.

The channel 111 through which the solvent sent from the solvent tank 2 of the anchor substrate 11 is in communication with the inlet 122 of the collection substrate 12 through a connecting tube 113 (see FIGS. 3A and 3B). The channel 112 through which the solvent sent to the hollow needle 6 of the anchor substrate 11 is in communication with the outlet 123 of the collection substrate 12 through the connecting tube 113. Preferably, the connecting tube 113 is anchored by being press fitted to part of the channel 111 and the channel 112, and be made of hard material (for example, metal) . The connecting tubes 113 fitted to the inlet 122 and the outlet 123 become solvent channels upon attaching the collection substrate 12 to the anchor substrate 11, and also serve as members for the registration of the collection substrate 12 on the anchor substrate 11.

It is desirable that a sealing member 13 be inserted between the anchor substrate 11 and the collection substrate 12 to prevent the solvent from leaking out of the surrounding area of the connecting tube 113 at the junction of the collection substrate 12 and the anchor substrate 11 attached in place. Preferably, elastic materials, such as silicon rubber, are used for the sealing member 13. Preferably, the sealing member 13 has a form of a sheet of about the same size as that of the collection substrate 12.

The material of the anchor substrate 11 and the collection substrate 12 may be, for example, glass material such as quartz and borosilicate glass, silicon rubber (such as polydimethylsiloxane; PDMS), acrylic resin (such as polymethylmethacrylate; PMMA), cycloolefin copolymer (COC), or polyetheretherketone (PEEK). The channels and other elements arranged on the substrate may be molded by the wet or dry etching of a glass substrate layer, or may be formed by the nanoimprinting, injection molding, or machining of a plastic substrate layer. It is preferable that the surface of the channels and other elements be subjected to a treatment that makes the adsorption of the physiologically active substance difficult. Such surface treatment may be performed using, for example, 2-methacryloyoxyethyl phosphorylcholine (MPC), and polyethylene glycol (PEG). Preferably, the collection substrate 12 is disposable.

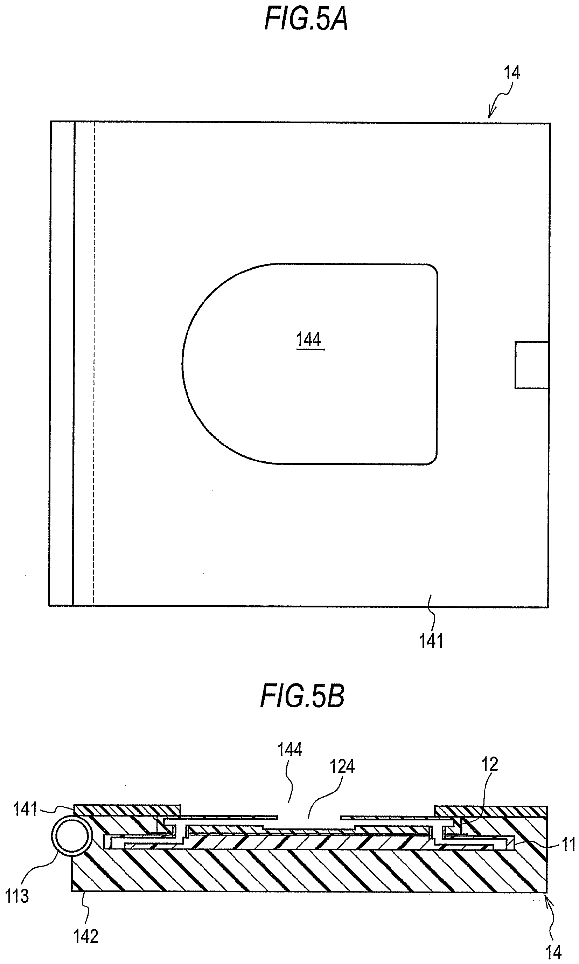

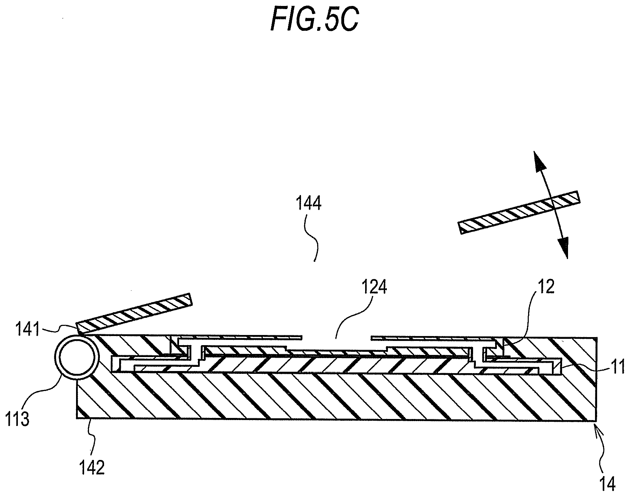

FIGS. 5A to 5C are schematic views explaining the configuration and operation of a holder used to hold the collection substrate 12 attached to the anchor substrate 11. FIG. 5A represents a top view, and FIGS. 5B and 5C represent cross sectional views.

As illustrated in the figures, a holder 14 includes an upper plate 141, a lower plate 142, and a hinge 143 that joins these plates. The upper plate 141 has a window 144 in a portion corresponding to the aperture 124 of the collection substrate 12 held by the holder 14. For the replacement of the collection substrate 12, the upper plate 141 is configured to open and close in the direction of arrow, using the hinge 143 as a fulcrum (see FIG. 5C).

The anchor substrate 11 is anchored to the main body of the device with the lower plate 142 of the holder 14. With the anchor substrate 11 and the collection substrate 12 sandwiched between the upper plate 141 and the lower plate 142 of the holder 14, the upper plate 141 presses down the collection substrate 12 attached to the anchor substrate 11. In this way, the holder 14 tightly holds the anchor substrate 11 and the collection substrate 12 via the sealing member 13 placed between the two substrates (see FIG. 4), and thus prevents the solvent from leaking out of the surrounding area of the connecting tube 113.

### (4) Collecting Unit

The operation of the collecting unit including the hollow needle 6 is described below with reference to FIGS. 6A and 6B, and FIG. 7.

The hollow needle 6 is supported by a needle plate 63. The needle plate 63 is movable in the positive and negative directions (upward and downward) along the Z axis in the figure with a driving unit realized by, for example, a feed screw, a guide, and a motor.

Referring to FIGS. 6A and 6B, when the hollow needle 6 is moved upward and the needle end out of the container 5, the solvent drained through the hole at the needle end is collected into the waste liquid tray 7, and sent to the waste liquid tank 4 for storage.

As illustrated in FIG. 7, the hollow needle 6 is moved downward for the injection of the solvent into the container 5 after the solvent has contacted the body surface and is sent from the collecting section 1. Here, the waste liquid tray 7 is moved in the positive direction along the X axis with a driving unit realized by, for example, a feed screw, a guide, and a motor, and retreats from between the hollow needle 6 and the container 5 disposed underneath, in order not to interfere with the downward movement of the hollow needle 6.

The waste liquid tray 7 has a notch 71 that provides a passage way for the hollow needle 6 during the positive-direction movement along the X axis. The notch 71 is formed by cutting out a part of the side wall of the waste liquid tray 7 on the side of the hollow needle 6 in a width greater than the thickness of the hollow needle 6. The provision of the notch 71 allows the waste liquid tray 7 to retreat from between the hollow needle 6 and the container 5 disposed underneath, only by moving in the X axis direction (horizontal direction) during the downward movement of the hollow needle 6.

With the hollow needle 6 down, the needle end is inserted into the container 5 disposed underneath, and the solvent drained through the hole at the needle end is introduced into the container 5. The container 5 is closed with a self-sealing sealant 51 that is elastic and undergoes elastic deformation. The hollow needle 6, on the other hand, has a sharp end that can penetrate through the sealant 51. Thus, the needle end penetrates through the sealant 51 into the container 5 as the hollow needle 6 moves downward. It is preferable that a groove that serves as an air evacuation channel be formed on the side surface of the hollow needle 6, so that the air inside the container 5 can be evacuated during the injection of the solvent into the container 5.

The hollow needle 6 is moved upward after the solvent is injected into the container 5. Here, the waste liquid tray 7 is in the retreat position not to interfere with the lifting of the hollow needle 6. Upon the hollow needle end being pulled out of the container 5 through the sealant 51 with the upward movement of the hollow needle 6, the portion penetrated by the needle seals itself by the resiliency of the sealant 51 undergoing elastic deformation. As used herein, this natural sealing of the penetrated portion by the elastic deformation of the sealant 51 is referred to as "self-sealing". The sealant 51 may be realized by, for example, synthetic rubber or natural rubber. Preferably, the sealant 51 is subjected to a surface treatment that makes the adsorption of the physiologically active substance difficult.

By the repeated upward and downward movement of the hollow needle 6, samples are successively injected into the containers 5 successively carried underneath by the conveyer chain. Note that the draining of the solvent through the hole at the needle end is controlled at appropriate timing with the valve 62 and the system control unit described in FIG. 2.

### (5) Quantifying Section and Determining Section

As described above, the physiologically active substance collecting device A functions to collect the physiologically active substance in the solvent by allowing the solvent to contact the body surface at the collecting section 1, and to collect the physiologically active substance in the container 5. In addition to the physiologically active substance acquiring function, the physiologically active substance collecting device A may additionally function to quantify the physiologically active substance and/or to automatically determine and acquire information concerning a living organism (biological information) based on a quantified value.

The quantifying section for the physiologically active substance can be configured using, for example, liquid chromatography (HPLC), a surface plasmon sensor (SPR), or a quarts crystal microbalance sensor (QCM) that quantifies a part of or all of the physiologically active substance collected at the collecting section 1. The quantifying section also may be configured to measure physiologically active substances based on known techniques, such as enzyme immunoassay, and radioimmunoassay.

HPLC, SPR, and QCM do not require the labeling required in enzyme immunoassay and radioimmunoassay, and thus simplify the configuration of the quantifying section. Use of SPR or QCM is more desirable in terms of measurement accuracy. In HPLC, physiologically active substances are detected as peaks on a chromatograph, and thus inclusion of foreign substance signals or noise in the peak intensity may lower measurement accuracy. On the other hand, SPR and QCM detect physiologically active substances using antibodies immobilized on the sensor surface, and thus can have high measurement accuracy based on antibody specificity. Another advantage of SPR and QCM over HPLC includes higher throughputs, and smaller quantifying sections.

The biological information determining section may be configured from analyzers and displays used in common HPLC, SPR, and QCM. The determining section acquires biological information using the quantified value of the quantifying section as an index. Specifically, for example, the amounts of physiologically active substances in large numbers of healthy subjects over a predetermined time period in a day are measured, and a standard change curve that defines a standard range of concentration changes of physiologically active substance amounts is calculated based on the measurement result. The amount of physiologically active substance in a subject is then compared with the standard change curve to determine biological information. The result of determination is then displayed on a display or the like.

The physiologically active substance collecting device A described above can be used to acquire a physiologically active substance from the body surface such as finger and palm surface, and can thus collect physiologically active substances in a simpler, more minimally invasive fashion than in methods that collect physiologically active substances from blood, urine, or saliva. Further, because the physiologically active substance acquired is that that is present on the body surface, the physiologically active substance can be acquired without making the subject strongly aware of the collection procedure, unlike the collection from bodily fluids such as blood, urine, and saliva. Thus, the physiologically active substance collecting device A can be used to collect the physiologically active substance, and acquire accurate biological information from the quantified value of the collected physiologically active substance, without causing stress or emotional changes in the subject.

Further, because the physiologically active substance collecting device A acquires the physiologically active substance secreted or permeated to the body surface, the physiologically active substance can be collected over a time course or on a steady basis. Further, because the physiologically active substance secreted or permeated to the body surface is collected, the physiologically active substance can be collected while the physiologically active substance is being metabolized in the body. The physiologically active substance collecting device A can thus be used to acquire the physiologically active substance from the subject over a time course or on a steady basis, and thus enables real-time sensing of biological information from the quantified value.

### 2. Physiologically Active Substance Collecting Device According to Variations of First Embodiment

The physiologically active substance collecting device A according to First Embodiment has been described through the collecting section 1 formed by attaching the collection substrate 12 with the holder 14 to the anchor substrate 11 anchored on the device main body. However, the configuration of the collecting section 1 may be varied as follows.

### (1) First Variation

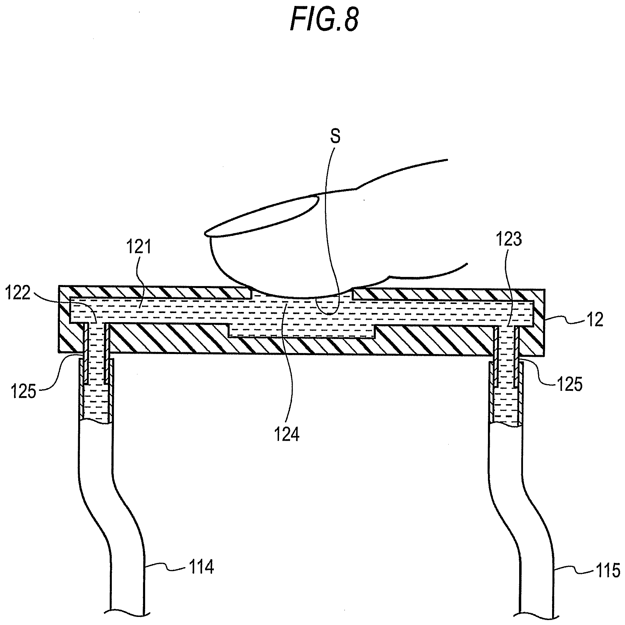

FIG. 8 is a schematic view explaining the configuration of a variation of the collecting section provided in the physiologically active substance collecting device according to the embodiment of the present disclosure. The figure represents the procedure of acquiring a physiologically active substance from the body surface at the collecting section. The collecting section according to the present variation is configured to enable the collection of a physiologically active substance from the body surface S with the collection substrate 12 separated from the anchor substrate 11 and from the main body of the device.

The collection substrate 12 is connected to the anchor substrate 11 (not illustrated) via tubes 114 and 115. The solvent (and air) sent from the anchor substrate 11 side is introduced to the channel 121 through the tube 114. After contacting the body surface S at the aperture 124, the solvent is sent to the anchor substrate 11 side through the tube 115. In FIG. 8, slots 125 are fitted to the inlet 122 and the outlet 123 of the channel 121, and connected to the tubes 114 and 115. The slots 125 may be metal or plastic tubes.

The collecting section according to the present variation is configured to include the collection substrate 12 separately provided from the device main body. Thus, by appropriately setting the length of the tubes 114 and 115, for example, the procedure of pressing a finger tip against the collection substrate 12 can be made with the collection substrate 12 brought close to the hand. Alternatively, the collection substrate 12 may be attached to the skin surface of the trunk to collect a physiologically active substance. When collecting a physiologically active substance from the skin surface of the trunk, the collection substrate 12 may be attached to the skin using an adhesive tape, or by wrapping a band around the trunk.

### (2) Second Variation

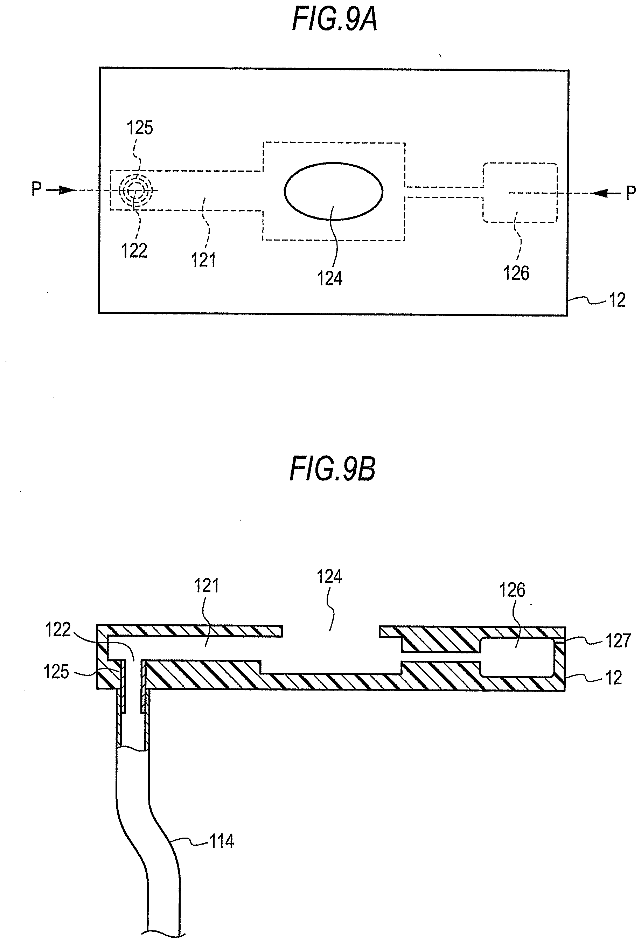

FIGS. 9A and 9B are schematic views explaining the configuration of another variation of the collecting section provided in the physiologically active substance collecting device according to the embodiment of the present disclosure. FIG. 9A represents a top view, and FIG. 9B a cross sectional view taken at P-P in FIG. 9A. The collecting section according to the present variation includes a collection region 126 formed in the collection substrate 12, and that stores the solvent that has contacted the body surface at the aperture 124.

The slot 125 is provided at the inlet 122 of the collection substrate 12, and is connected to the tube 114 through which the solvent sent from the anchor substrate 11 is introduced to the channel 121. The solvent introduced to the channel 121 and contacted the body surface at the aperture 124 is introduced to the collection region 126 and stored therein. In the figures, an air vent 127 is provided through which the air inside the collection region 126 is evacuated by being pushed by the introduced solvent.

The collecting section according to the present variation is configured to store a sample in the collection region 126 internally provided for the collection substrate 12, and is therefore suited for a single or a few collections of samples. Cross contamination between samples can be avoided by replacing the collection substrate 12 for each sample collection. Further, samples can be collected more easily, because the use of the collecting unit including the hollow needle 6 is not necessary.

The collection region 126 and the air vent 127 can be molded in the collection substrate 12 by the wet or dry etching of a glass substrate layer, or by the nanoimprinting, injection molding, or machining of a plastic substrate layer. More than one collection region 126 maybe provided. In this case, the channel 121 downstream of the aperture 124 is branched and connected to each collection region 126. The branching portion of the channel 121 into the collection regions 126 may be provided with a switch valve that sends the solvent to one of the collection regions 126.

### (3) Third Variation

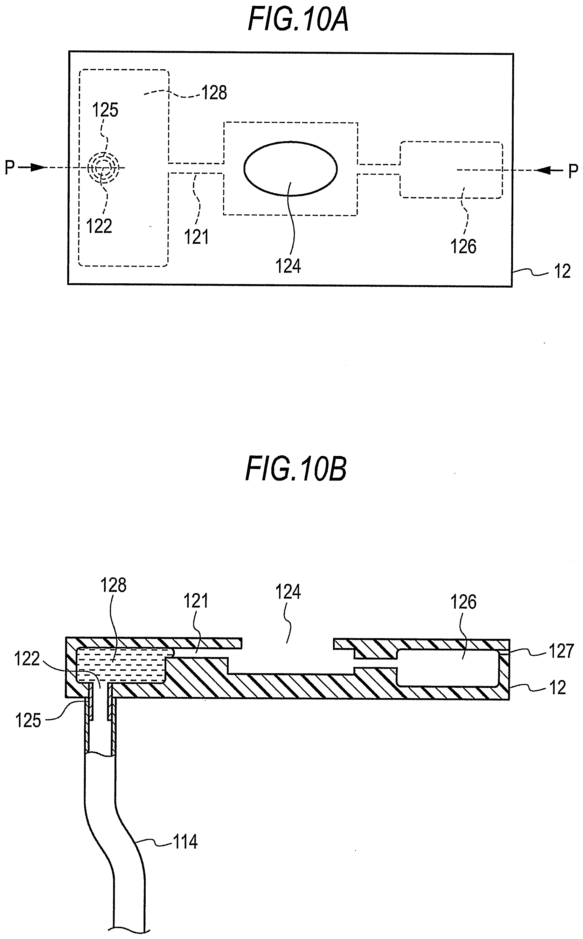

FIGS. 10A and 10B are schematic views explaining the configuration of yet another variation of the collecting section provided in the physiologically active substance collecting device according to the embodiment of the present disclosure. FIG. 10A represents a top view, and FIG. 10B a cross sectional view taken at P-P in FIG. 10A. The collecting section according to the present variation includes a solvent storage region 128 formed in the collection substrate 12, and that can store the solvent for later use.

The solvent required for a single or a few sample collections can be injected to the solvent storage region 128 and stored therein in advance. The slot 125 is provided at the inlet 122 of the collection substrate 12, and is connected to the tube 114 through which the air sent from the anchor substrate 11 is introduced to the channel 121. The air introduced to the channel 121 pushes the solvent stored beforehand in the solvent storage region 128. The solvent then contacts the body surface at the aperture 124, and is introduced to the collection region 126 and stored therein.

The collecting section according to the present variation enables sample collection with the use of a solvent stored beforehand in the solvent storage region 128 internally provided for the collection substrate 12, and is therefore preferred for sample collections in which the solvent is changed for each sample.

The solvent storage region 128 can be molded in the collection substrate 12 by the wet or dry etching of a glass substrate layer, or by the nanoimprinting, injection molding, or machining of a plastic substrate layer.

### 3. Physiologically Active Substance and Biological Information

Examples of the biological information acquired using the physiologically active substance collecting device according to the embodiment of the present disclosure include information concerning stress, emotion, menstrual cycle, and exercise effects. Other examples include sleepiness (wakefulness level), health conditions, and circadian rhythm (biological rhythm).

Concerning stress, there is a well known correlation between the stress load on a living organism and the secretion levels of cortisol, corticosterone, and cortisone (hereinafter, collectively referred to as "cortisols"), as described in Patent Documents 3 and 4. As used herein, "secretion levels" are the secretion levels in blood; specifically, the term has the same meaning as "blood concentration".

Concerning emotion such as excitement, fear, anger, aggression, comfort, anxiety, and sorrow, there is a known correlation with the secretion levels of norepinephrine, epinephrine, dopamine, and L-DOPA, a precursor substance of these (hereinafter, collectively referred to as "catecholamines"). The correlation between emotion and the secretion levels of serotonin, a member of monoamines as are catecholamines, has also been elucidated.

For example, there is a report that the noradrenaline levels in saliva are different before and after a psychosocial test performed to give anxiety or fear to a subject (see Study of salivary catecholamines using fully automated column-switching high-performance liquid chromatography, Journal of Chromatography. B, Biomedical Sciences and Applications,. 1997 Jul 4; 694 (2): 305-16).

Further, as is well known, estrone (E1), estradiol (E2), and estriol (E3) (hereinafter, collectively referred to as "estrogens") control the menstrual cycle of a living organism, and their secretion levels vary in correlation with the menstrual cycle.

It is also known that effective exercises promote secretion of growth hormone. The secretion of growth hormone promotes muscle and bone growth, and facilitates the recruitment of body fat to increase fat combustion efficiency. It is therefore believed that the effects of exercises such as in muscle enhancement and dieting have a correlation with the secretion levels of growth hormone.

Thus, for example, the quantified value of cortisols can be used to obtain information concerning the stress placed on a living organism. Specifically, for example, the secretion levels of cortisols in large numbers of healthy subjects are measured, and a standard change curve that defines a standard range of concentration changes of cortisols is calculated based on the measurement result. The secretion levels of cortisols in a subject are then measured, and the result is compared with the standard change curve. For example, if the measured secretion levels deviate from the standard change curve, it can be determined that the subject is under chronic stress.

Further, for example, the secretion levels of cortisols in a subject under normal conditions are measured, and a standard change curve is calculated from the measurement results. The standard change curve can then be compared with the secretion levels of cortisols in the subject at a given time to determine whether the subject at the given point of time is under stress or relaxing.

Aside from the cortisols, monoamines, estrogens and growth hormones, the combinations of index physiologically active substances and biological information presented in Table 1 are known. In the embodiment of the present disclosure, by using these combinations, information concerning biological information can be obtained based on the positive or negative correlation between the quantified value of the physiologically active substance and biological information.

**Table 1**

| Biological information | Physiologically active substance | |
|---|---|---|
| Stress | Steroid hormones | Cortisol, corticosterone, cortisone |
| | Peptides | Neuropeptide Y (NPY) |
| Emotion (aggression) | Steroid hormones | Testosterone, dihydrotestosterone (DHT), dehydroepiandrosterone (DHEA), dehydroepiandrosterone sulfate (DHEAS) |
| Emotion (excitement, fear, anger, etc.) | Monoamines (catecholamines) | Noradrenaline (norepinephrine), adrenaline (epinephrine), L-DOPA |
| Emotion (comfort) | Monoamines (catecholamines) | Dopamine |
| | Peptides | Endorphin |
| Emotion (anxiety) | Monoamines | Serotonin |
| | Peptides | Oxytocin, vasopressin, galanin |
| Sleepiness (wakefulness level) | | Melatonin |
| Menstrual cycle | Steroid hormones | Estrone (E1), estradiol (E2), estriol (E3) |

Note that the physiologically active substances presented in Table 1 are merely examples, and other catecholamines, for example, such as metanephrine, normetanephrine, 3-methoxy-4-hydroxymandelic acid, 3-methoxy-4-hydroxyphenylglycol, 3,4-dihydroxymandelic acid, 3,4-dihydroxyphenylglycol, 3,4-dihydroxyphenylacetic acid, 3-methoxytyramin, homovanillic acid, 5-hydroxyindoleacetic acid, and vanillylmandelic acid also can be used as the index of biological information. Other examples of steroid hormones that also can be used as the index of biological information include aldosterone, deoxycortisterone, androstenedione, progesterone, 11-deoxycorticosterone, pregnenolone, 11-deoxycortisol, 17-hydroxyprogesterone, 17-hydroxypregnenolone, and cholecalciferol (vitamin D).

Other examples of physiologically active substances that also can be used as the index of biological information include: hypophysiotropic hormones such as corticotropin release hormone (CRH), growth hormone release hormone (GRH), somatostatin (growth hormone secretion inhibiting hormone), gonadotropin release hormone (GnRH), prolactin release hormone (PRH), prolactin inhibiting hormone (PIH), thyrotropin release hormone (TRH), and thyroid-stimulating hormone (TSH); thyroid hormones such as thyroxine and triiodothyronine; and various other hormones and neurotransmitters, including chromogranin A, adrenocorticotropic hormone (ACTH), luteinizing hormone (LH), insulin-like growth factor I (IGF-I), prolactin, proopiomelanocortin (POMC), oxytocin, α-melanocyte stimulating hormone (α-MSH), glucagon, ghrelin, galanin, motilin, leptin, gastrin, cholecystokinin, selectin, activin, inhibin, neurotensin, bombesin, substance P, angiotensin I, II, enkephalin, orexinA, B, anandamide, acetylcholine, histamine, glutamic acid, glycine, aspartic acid, pyrimidine, adenosine, adenosine triphosphate (ATP), GABA, FMRF amide, peptide YY, Agouti-related peptide (AGRP), cocaine- and amphetamine-regulated transcript (CART), calcitonin gene-related peptide (CGRP), glucagon-like peptide 1, 2 (GLP-1, 2), vasoactive intestinal peptide (VIP), gastrin release peptide (GRP), and melanin-concentrating hormone (MCH).

The correspondence between the physiologically active substances and biological information is not limited to the foregoing examples. For example, serotonin also can be used as an index of schizophrenia or insomnia as for emotions, and estrogens also can be used as an index of infertility, symptoms of menopause, or a manic-depressive state as for the menstrual cycle. In fact, the combination of the physiologically active substance and the corresponding biological information may be any combination elucidated to date.

The physiologically active substance collecting device according to the embodiment of the present disclosure can be used for the diagnosis, prevention, or prognostic study of various diseases based on the health conditions of a living organism found by using, for example, the physiologically active substances presented in Table 1 as an index. Specifically, for example, a diagnosis can be made to find the presence or absence of chronic stress through the measurement of cortisols, and the diagnosis result can be used for the prevention or prognostic study of chronic stress. It is also considered possible to make, for example, a diagnosis for the presence or absence of a carcinoid tumor through the measurement of catecholamines, or a diagnosis for schizophrenia, insomnia, endogenous depression, dumping syndrome, or migraine through the measurement of serotonin. Further, estrogens can be measured for easy diagnosis of menstrual cycle, and for the diagnosis of estrogen-dependent diseases (such as infertility, breast cancer, uterine fibroid, and endometriosis), and symptoms of menopause.

The secretion levels of growth hormone is known to decrease with age. Involvement of growth hormone in the onset of lifestyle-related disease such as diabetes, high-blood pressure, and hyperlipidemia through its action on the metabolism of carbohydrates, proteins, and lipids is also known. Other examples of growth hormone-related disease include growth hormone deficiency, hypopituitarism, hypothyroidism, and obesity, which involve decreased secretion levels of growth hormone. Other examples include gigantism, acromegaly, ectopic hormone-producing tumor, severe undernutrition (such as anorexia nervosa), and chronic kidney failure, which involve increased secretion levels of growth hormone. Thus, the growth hormone measurement by the physiologically active substance measurement method according to the embodiment of the present disclosure allows the extent of aging to be determined, and enables the diagnosis of various diseases, including lifestyle-related disease and growth hormone-related disease.

### Examples

### Example 1: Quantification of Cortisols

### 1. Acquisition of Cortisol from Skin Surface

Cortisol was acquired from the skin surface of the fingers of six subjects, three times a day (10, 14, 18 o'clock) for 4 days, using the two methods below.

### (1) Collection Using Microtube

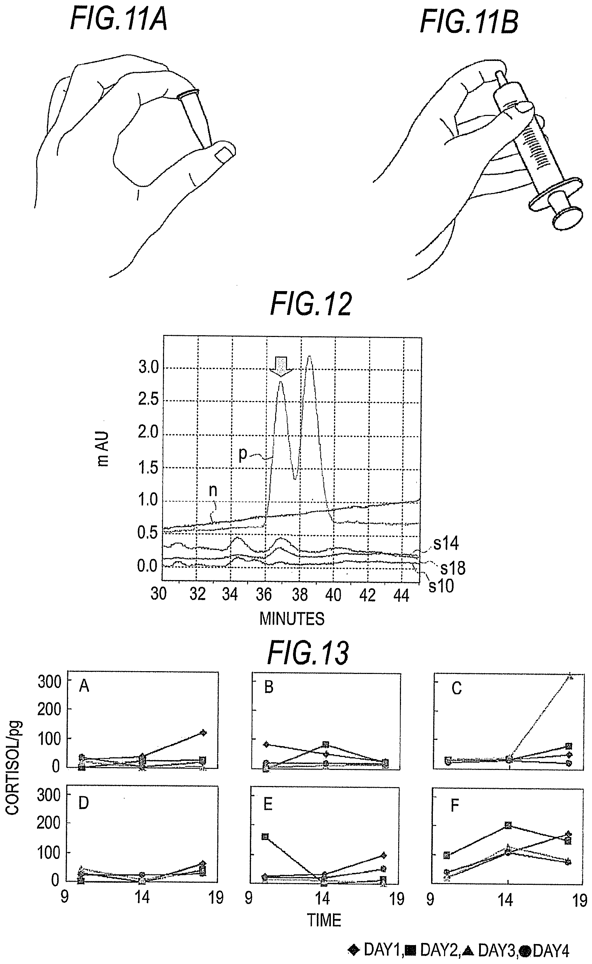

The finger tip of index finger was gently wiped with a paper towel soaked with ethanol. The lower end of a microtube containing 1% ethanol water (50 µL) was held with the thumb, with the upper opening touching the finger tip of the index finger (see FIG. 11A) . The microtube was inverted between the index finger and thumb to contact the 1% ethanol water to the skin surface of the index finger for 1 min. Here, the finger tip was wiped in advance with a paper towel to remove foreign substances present on the skin surface, and to remove the possible accumulation of cortisol on the skin surface.

### (2) Collection Using Syringe

The finger tip of index finger was gently wiped with a paper towel soaked with ethanol. After charging 1% ethanol water (50 µL) into the tip of a syringe, the syringe was held with the thumb and middle finger, with the tip of the index finger touching the syringe (see FIG. 11B) . The piston of the syringe was then pulled with the right hand to create a negative pressure therein and suck the skin surface, and to thereby contact the 1% ethanol water to the skin surface of the index finger for 1 min. This method is more advantageous than the collection using a microtube described in (1) above, because the method enables the 1% ethanol water contacted to the skin surface to be collected in higher yield based on the negative pressure in the syringe.

### 2. Quantification Using High-Performance Liquid Chromatography (HPLC)

40 µL of 1% ethanol water contacted to skin surface (hereinafter, simply "sample") was collected in a vial. 30 µL of the sample was then analyzed by high-performance liquid chromatography (NANOSPACE SI-2, SHISEIDO).

2.5% acetonitrile water was flown at a flow rate of 100 µL/min, using CAPCELLPAK MF Ph-1 (column size 1.5 mm ID × 35 mm, column temperature 35°C, SHISEIDO) as a pretreatment column. 10 mM phosphate buffer (pH 6.8)/CH₃CN = 78/22 was flown at a flow rate of 100 µL/min using CAPCELLPAK C18 UG120 (column size 1.5 mm ID × 250 mm, column temperature 35°C, SHISEIDO) as an analytical column. An ultraviolet absorbance detector (wavelength 242 nm UV) was used for the detection, and the measurement was made for 50 min.

Standard cortisol (Wako Pure Chemical Industries, Ltd.) was prepared as a 0.5 µM cortisol/cortisone aqueous solution, and a preliminary study was made. In the preliminary study, the valve switching time from the pretreatment column to the analytical column (2.7 to 4.4 min from the start of measurement), and the cortisol efflux time (36 to 38 min from the same reference point) were confirmed.

FIG. 12 represents the measurement results of the cortisol levels in samples collected from one subject three times a day (10, 14, 18 o'clock). In the figure, cortisol peaks can be confirmed in the samples collected at each time (s10, s14, s18). As indicated by block arrow, the peaks correspond to the standard peak p. Note that, the measurement result for 1% ethanol water that was not contacted to the skin is indicated by n.

FIG. 13 in A to F represents the cortisol levels (pg) calculated from the peak area determined based on the baseline, using the standard curve. The results represented in FIG. 13 are the results of the measurements performed for six subjects (subjects A to F) for 4 days. The results confirmed that cortisol could be collected from the skin surface in amounts ranging from several picograms to as high as 300 picograms, though the results varied from one individual to another, and depending on the measurement time.

### 3. Quantification Using Surface Plasmon Sensor (SPR)

The samples prepared using the method of Example 1 (collection by syringe) were analyzed by indirect competitive SPR using a surface plasmon sensor (Biacore X, Biacore). The analysis was performed according to the following procedure.

### (1) Immobilization of Cortisol on SPR Sensor Surface

An SA chip (Biacore) including streptavidin pre-immobilized on surface was used as the SPR sensor. Standard cortisol was biotinylated, and, after being dissolved in Acetate 4.0 (Biacore), injected at a flow rate of 10 µL/min (100 µL) to immobilize the cortisol on the SPR sensor surface through avidin-biotin reaction. The immobilized cortisol was about 150 RU.

### (2) Creation of Standard Curve

First, standard cortisol as a 10 mM DMSO (dimethyl sulfoxide) solution was serially diluted using 1% ethanol water to prepare 100, 50, 25, 12.5, 6.25, 3.13, 1.56, and 0.78 nM standard solutions. 40 µL of the standard solution of each concentration was thoroughly mixed with 40 µL of a 5 ng/mL anti-cortisol antibody solution to run a binding reaction. After the binding reaction, 25 µL of the standard sample solution was injected at 10 µL/min, at 25°C. Note that mouse monoclonal antibodies (XM210; Abcam) were used as the anti-cortisol antibodies, and HBS-EP buffer (Biacore) as the running buffer.

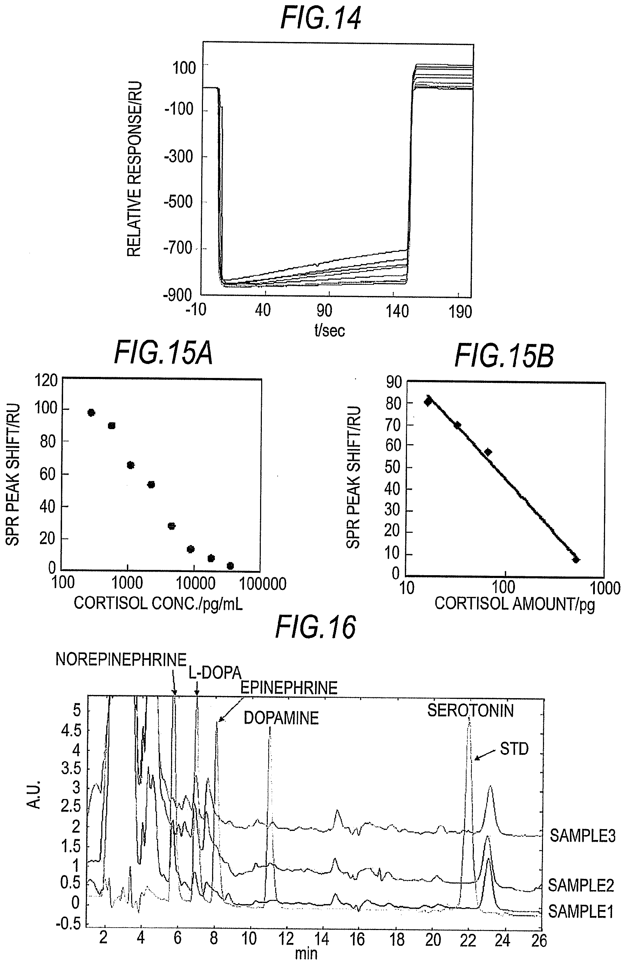

FIG. 14 represents the SPR curve obtained for the cortisol solution of each concentration. In the figure, the peak shift of about 850 RU (Resonance Unit) occurring at 0 sec is the bulk effect due to the switching from the running buffer to the standard sample solution. The bulk effect disappears at 150 sec by the switching of the standard sample solution to the running buffer.

From 0 to 150 sec, a time-course increase of RU was observed as a result of the binding of the anti-cortisol antibodies to the cortisol immobilized on the surface of the sensor substrate. The RU increase was smaller in higher concentrations of the standard cortisol solution, and larger in lower concentrations of the standard cortisol solution. This suggests that the indirect competitive SPR has functioned according to its measurement principle.

FIG. 15A is a graph obtained by calculating RU 60 seconds after the end of the injection in comparison to the baseline.

### (3) Sample Measurement

40 µL of the sample prepared in Example 1 was thoroughly mixed with 40 µL of an anti-cortisol antibody solution to run a binding reaction. After the binding reaction, 40 µL of the standard sample solution was injected at 20 µL/min, at 25°C. The standard curve created under the same conditions is represented in FIG. 15B.

Table 2 presents cortisol levels (pg) calculated for eight subjects (subjects a to h) using the standard curve. Several ten picograms of cortisol were detected in each subject.

**Table 2**

| Subject | Response/RU | Cortisol/pg |
|---|---|---|
| a | 72.5 | 27.10 |
| b | 91.5 | 11.09 |
| c | 53.8 | 65.33 |
| d | 76.4 | 22.56 |
| e | 74.7 | 24.44 |
| f | 68.3 | 33.02 |
| g | 57.2 | 55.67 |
| h | 47.6 | 87.46 |

### Example 2: Quantification of Catecholamines

### 1. Acquisition of Norepinephrine and L-DOPA from Skin Surface

Norepinephrine and L-DOPA were collected from the skin surface according to (1) the collection method using a microtube described in Section 1 (Acquisition of Cortisol from Skin Surface) of Example 1. In this Example, however, water was used as the solvent, and the contact time for the skin surface was 3 min.

### 2. Quantification Using High-Performance Liquid Chromatography (HPLC)

40 µL of water contacted to the skin surface (hereinafter, simply "sample") was collected in a vial. 30 µL of the sample was then analyzed by high-performance liquid chromatography (NANOSPACE SI-2, SHISEIDO).

2.5% acetonitrile water was flown at a flow rate of 100 µL/min, using CAPCELLPAK MF Ph-1 (column size 1.5 mm ID × 35 mm, column temperature 35°C, SHISEIDO) as a pretreatment column. CAPCELLPAK C18 MGII S5 (column size 2.0 mm I.D. × 250 mm, column temperature 40°C, SHISEIDO) was used as an analytical column.
Mobile phase: A/B = 90/10 ((A) 1.0 mM sodium octanesulfonate, 0.02 mM EDTA-2Na, 10 mM KH₂PO₄, 0.05 vol% H₃PO₄, (B) CH₃CN)
Flow rate: 200 µL
Injection amount: 2 µL or 5 µL

The measurement results are presented in FIG. 16. Sample-1 represents a chromatogram obtained from a concentrated solution of the sample. Sample-2 and Sample-3 represent chromatograms obtained from unconcentrated samples. STD represents a chromatogram obtained from a standard solution (a solution containing norepinephrine, epinephrine, L-DOPA, dopamine, and serotonin).

While peaks corresponding to norepinephrine and L-DOPA were detected in the chromatograms of Sample-1 to Sample-3, no peaks were detected that corresponded to epinephrine, dopamine, and serotonin.

Table 3 presents the norepinephrine and L-DOPA levels (pg) calculated from the peak area determined based on the baseline, using the standard curve.

**Table 3**

| | Injection amount | | | |
|---|---|---|---|---|
| | 2 µl | 5 µl | | |
| | Norepinephrine | L-DOPA | Norepinephrine | L-DOPA |
| Sample-1 | 7.58 | 2.42 | 19.37 | 7.17 |
| Sample-2 | N.D. | 5.50 | N.D. | 15.68 |
| Sample-3 | N.D. | 5.35 | N.D. | 14.71 |

Norepinephrine was below the detection limit (N.D.) in the unconcentrated Sample-2 and Sample-3, whereas about several to several ten picograms were detected in the concentrated Sample-1. About several to several ten picograms of L-DOPA were quantified in all of Sample-1 to Sample-3. The results thus confirmed that about several to several ten picograms of norepinephrine and L-DOPA could be collected from the skin surface.

### Example 3: Quantification of Serotonin

### 1. Acquisition of Serotonin from Skin Surface

Serotonin was collected from the skin surface according to the method of Example 2.

### 2. Quantification Using High-Performance Liquid Chromatography (HPLC)

100 µL of water contacted to skin surface (hereinafter, simply "sample") was collected in a vial. 100 µL of the sample was then analyzed by high-performance liquid chromatography (NANOSPACE SI-2, SHISEIDO).

CAPCELL PAK C18 MGII S5 (column size 2.0 mm ID × 35 mm, column temperature 40°C, SHISEIDO) was used as a pretreatment column. CAPCELLPAK C18 UG120 S3 (column size 1.5 mm ID × 250 mm, column temperature 40°C, SHISEIDO) was used as an analytical column. Mobile phase (A/B = 87/13 ((A) 4 mM Sodium 1-octanesulfonate, 0.02 mM EDTA-2Na, 5 mM KH₂PO₄ (pH 3.4); (B) CH₃CN)) was fed at a flow rate of 100 µL/min. An electrochemical detector (ECD OX 750mV (Ag) was used for the detection.

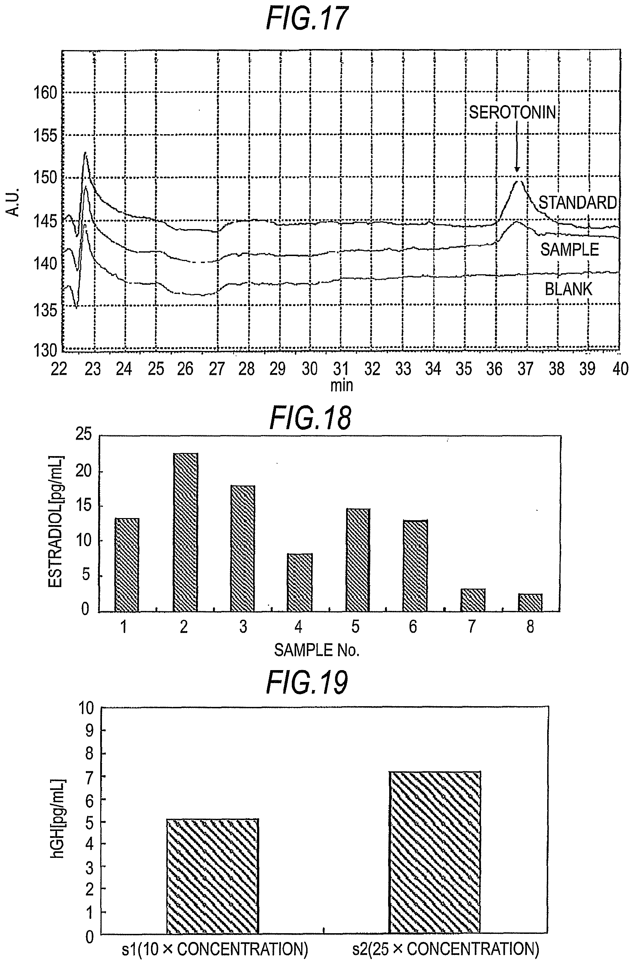

The measurement results are presented in FIG. 17. "Sample" represents a chromatogram obtained from a concentrated solution (100×) of the sample. "Standard" represents a chromatogram obtained from a 0.1 µM solution of standard serotonin (Wako Pure Chemical Industries, Ltd.).

In the sample and standard serotonin chromatograms, peaks are detected at the elution time of 36 to 38 min. The serotonin concentration calculated from the area in the chromatogram was about 4.4 ng/mL in the 100X concentrated solution of the sample, and about 0.044 ng/mL of serotonin were collected from the skin surface of the index finger.

### Example 4: Quantification of Estradiol

### 1. Acquisition of Estradiol from Skin Surface

Estradiol was collected from skin surface according to the method of Example 2. Estradiol was collected from eight subjects.

### 2. Quantification Using Enzyme Immunoassay (Enzyme-Linked Immunosorbent Assay: ELISA)

100 µL of water contacted to skin surface (hereinafter, simply "sample") was collected in a vial. 100 µL of the sample was then analyzed using a commercially available ELISA kit (High Sensitivity SALIVARY 17β-ESTRADIOL ENZYME IMMNOASSAY KIT, SALIMETRICS).

A standard curve was created through the measurement of the standard estradiol attached to the kit, and the estradiol concentration in the sample was calculated. The results are presented in FIG. 18. The estradiol concentration was about 2 to 23 pg/ml in each sample. The results thus confirmed that about several to several ten picograms of estradiol could be collected from the skin surface of the index finger.

### Example 5: Quantification of Growth Hormone

### 1. Acquisition of Growth Hormone from Skin Surface

Growth hormone was collected from skin surface according to the method of Example 2. Here, growth hormone was collected from the skin surface of the thumbs of three subjects.

### 2. Quantification Using Enzyme Immunoassay (ELISA)

100 µL of the sample was collected in a vial, and analyzed using a commercially available ELISA kit (hGH ELISA, Roche).

A standard curve was created through the measurement of the standard growth hormone attached to the kit, and the growth hormone concentration in the sample was calculated. The results are presented in FIG. 19. In the figure, s1 is the 10× concentrated solution, and s2 the 25× concentrated solution of the sample. It was possible to collect 0.29 to 0.51 pg/mL of growth hormone from the skin surface of the thumb.

The physiologically active substance collecting device according to the embodiment of the present disclosure can be used to collect a physiologically active substance from a living organism on a steady basis in a convenient and minimally invasive manner. Because the device can acquire accurate biological information based on the quantified value of the acquired physiologically active substance, the present disclosure can be used, for example, in biological information sensing in the fields of home healthcare and entertainment such as in games.

Although particular embodiments have been described herein, it will be appreciated that the invention is not limited thereto and that many modifications and additions thereto may be made within the scope of the invention. For example, various combinations of the features of the following dependent claims can be made with the features of the independent claims without departing from the scope of the present invention.

## Claims

1. A physiologically active substance collecting device, comprising:
a collecting section (1) configured to be brought into contact with a body surface of a living organism to acquire a physiologically active substance from the body surface; and
a liquid sending means (2; 21; 22) for sending a solvent to the collecting section via an inlet (122),
the collecting section having an aperture (124) at which the solvent sent from the liquid sending means flows when the device is in use to contact the body surface, wherein the device is **characterised by** an air sending unit (3; 31; 32) configured to send air to the collecting section via the inlet to selectively introduce the solvent and air into the collecting section.

2. The physiologically active substance collecting device according to claim 1, further comprising:
a collecting unit configured such that the solvent contacted the body surface at the aperture is injected into a container (5) sealed by an elastic, self-sealing sealant (51) that undergoes elastic deformation, wherein the collecting unit includes a hollow needle (6) capable of penetrating through the sealant, and draining the solvent through the hole of the hollow needle at a needle end.

3. The physiologically active substance collecting device according to claim 2, further comprising:
a first driving unit configured to move the hollow needle downward to place the needle end inside the container through the sealant, and upward to pull back the needle end out of the container through the sealant; and
a feeder configured to place the container one by one underneath the hollow needle.

4. The physiologically active substance collecting device according to claim 3, further comprising:
a waste liquid receptor (7) positioned between the hollow needle and the container when the needle end of the hollow needle is out of the container, so as to receive the solvent drained through the hole; and
a second driving unit configured to retreat the waste liquid receptor from between the hollow needle and the container in advance of the hollow needle moving downward.

5. The physiologically active substance collecting device according to claim 1,
wherein the collecting section includes a substrate (12) that includes a channel (112) configured to flow the solvent, a solvent inlet (122) for the channel, a solvent outlet (123) for the channel, and the aperture positioned between the inlet and the outlet of the channel, and
wherein the substrate is replaceable from the collecting section.

6. The physiologically active substance collecting device according to claim 5, further comprising:
a quantifying section configured to quantify the physiologically active substance; and
a determining section configured to automatically determine and acquire information concerning the living organism based on the quantified value of the physiologically active substance.

7. The physiologically active substance collecting device according to claim 6, configured such that information concerning stress is acquired as the information concerning the living organism, using cortisols as the physiologically active substance.

8. The physiologically active substance collecting device according to claim 6, configured such that information concerning emotion is acquired as the information concerning the living organism, using monoamines as the physiologically active substance.

9. The physiologically active substance collecting device according to claim 6, configured such that information concerning menstrual cycle is acquired as the information concerning the living organism, using estrogens as the physiologically active substance.

10. The physiologically active substance collecting device according to claim 6, configured such that information concerning exercise effect is acquired as the information concerning the living organism, using growth hormone as the physiologically active substance

## Patentansprüche

1. Sammelvorrichtung für physiologisch aktiven Stoff, umfassend:
einen Sammelabschnitt (1), der dafür gestaltet ist, mit einer Körperoberfläche eines lebenden Organismus in Kontakt gebracht zu werden, um einen physiologisch aktiven Stoff von der Körperoberfläche aufzunehmen; und
eine Flüssigkeitsbeförderungseinrichtung (2; 21; 22) zum Befördern eines Lösungsmittels zu dem Sammelabschnitt über einen Einlass (122),
wobei der Sammelabschnitt eine Öffnung (124) aufweist, zu der das von die Beförderungseinrichtung beförderte Lösungsmittel fließt, wenn die Vorrichtung in Verwendung ist, um in Kontakt mit der Körperoberfläche zu stehen, wobei die Vorrichtung durch eine Luftbeförderungseinheit (3; 31; 32) gekennzeichnet ist, die dafür gestaltet ist, über den Einlass Luft zu dem Sammelabschnitt zu befördern, um selektiv das Lösungsmittel und Luft in den Sammelabschnitt einzuführen.

2. Sammelvorrichtung für physiologisch aktiven Stoff gemäß Anspruch 1, ferner umfassend:
eine Sammeleinheit, die so gestaltet ist, dass das mit der Körperoberfläche in Kontakt gebrachte Lösungsmittel an der Öffnung in einen Behälter (5) eingespritzt wird, der mit einem elastischen selbstabdichtenden Dichtmittel (51) abgedichtet ist, das elastische Verformung erfährt, wobei die Sammeleinheit eine Hohlnadel (6) aufweist, die fähig ist, das Dichtmittel zu durchdringen, und das Lösungsmittel durch das Loch der Hohlnadel an dem Nadelende abgelassen wird.

3. Sammelvorrichtung für physiologisch aktiven Stoff gemäß Anspruch 2, ferner umfassend:
eine erste Antriebeinheit, die dafür gestaltet ist, die Hohlnadel nach unten zu bewegen, um das Nadelende durch das Dichtmittel im Inneren des Behälters zu platzieren, und das Nadelende durch das Dichtmittel aus dem Behälter zurück nach oben zu ziehen; und
eine Zuführeinrichtung, die dafür gestaltet ist, den Behälter einen nach dem anderen unter der Hohlnadel zu platzieren.

4. Sammelvorrichtung für physiologisch aktiven Stoff gemäß Anspruch 3, ferner umfassend:
eine Abfallflüssigkeitsaufnahme (7), die zwischen der Hohlnadel und dem Behälter angeordnet ist, wenn sich das Nadelende der Hohlnadel außerhalb des Behälters befindet, um das Lösungsmittel aufzunehmen, das durch das Loch abgelassen wird; und
eine zweite Antriebeinheit, die dafür gestaltet ist, die Abfallflüssigkeitsaufnahme von zwischen der Hohlnadel und dem Behälter zurückzuziehen, bevor sich die Hohlnadel nach unten bewegt.

5. Sammelvorrichtung für physiologisch aktiven Stoff gemäß Anspruch 1,
wobei der Sammelabschnitt ein Substrat (12) aufweist, das einen Kanal (112), der zum Leiten des Lösungsmittels gestaltet ist, einen Lösungsmitteleinlass (122) für den Kanal, einen Lösungsmittelauslass (123) für den Kanal, und die Öffnung, die zwischen dem Einlass und dem Auslass des Kanals angeordnet ist, aufweist, und
wobei das Substrat aus dem Sammelabschnitt ersetzbar ist.

6. Sammelvorrichtung für physiologisch aktiven Stoff gemäß Anspruch 5, ferner umfassend:
einen Quantifizierungsabschnitt, der dafür gestaltet ist, den physiologisch aktiven Stoff zu quantifizieren; und
einen Bestimmungsabschnitt, der dafür gestaltet ist, Informationen über den lebenden Organismus auf der Grundlage des quantifizierten Werts des physiologisch aktiven Stoffs automatisch zu bestimmen und aufzunehmen.

7. Sammelvorrichtung für physiologisch aktiven Stoff gemäß Anspruch 6, dafür gestaltet, dass Informationen hinsichtlich Stress als die Information hinsichtlich des lebenden Organismus unter Verwendung von Cortisolen als dem physiologisch wirksamen Stoff aufgenommen werden.

8. Sammelvorrichtung für physiologisch aktiven Stoff gemäß Anspruch 6, dafür gestaltet, dass Informationen hinsichtlich Emotion als die Information hinsichtlich des lebenden Organismus unter Verwendung von Monoaminen als dem physiologisch wirksamen Stoff aufgenommen werden.

9. Sammelvorrichtung für physiologisch aktiven Stoff gemäß Anspruch 6, dafür gestaltet, dass Informationen hinsichtlich des Menstruationszyklus als die Information hinsichtlich des lebenden Organismus unter Verwendung von Estrogenen als dem physiologisch wirksamen Stoff aufgenommen werden.

10. Sammelvorrichtung für physiologisch aktiven Stoff gemäß Anspruch 6, dafür gestaltet, dass Informationen hinsichtlich der Wirkung körperlicher Betätigung als die Information hinsichtlich des lebenden Organismus unter Verwendung von Wachstumshormon als dem physiologisch wirksamen Stoff aufgenommen werden.

## Revendications

1. Dispositif de collecte de substance physiologiquement active, comprenant:
une section de collecte (1) configurée de manière à être amenée en contact avec une surface de corps d'un organisme vivant dans le but d'acquérir une substance physiologiquement active à partir de la surface de corps; et
des moyens d'envoi de liquide (2; 21; 22) pour envoyer un solvant à la section de collecte par l'intermédiaire d'une entrée (122),
la section de collecte comportant une ouverture (124) par laquelle le solvant envoyé par les moyens d'envoi de liquide s'écoule lorsque le dispositif est, lors de l'utilisation, mis en contact avec la surface de corps, dans lequel le dispositif est **caractérisé par** une unité d'envoi d'air (3; 31; 32) configurée de manière à envoyer de l'air à la station de collecte par l'intermédiaire de l'entrée afin d'introduire de façon sélective le solvant et l'air dans la section de collecte.

2. Dispositif de collecte de substance physiologiquement active selon la revendication 1, comprenant en outre une unité de collecte configurée de telle sorte que le solvant mis en contact avec la surface de corps au niveau de l'ouverture soit injecté dans un récipient (5) fermé par un matériau d'étanchéité élastique auto-obturant (51) qui subit une déformation élastique, dans lequel l'unité de collecte comprend une aiguille creuse (6) capable de pénétrer à travers le matériau d'étanchéité, et de drainer le solvant à travers le trou de l'aiguille creuse à une extrémité de l'aiguille.

3. Dispositif de collecte de substance physiologiquement active selon la revendication 2, comprenant en outre:
une première unité d'entraînement configurée de manière à déplacer l'aiguille creuse vers le bas afin de placer l'extrémité de l'aiguille à l'intérieur du récipient à travers le matériau d'étanchéité, et vers le haut afin de retirer l'extrémité de l'aiguille hors du récipient à travers le matériau d'étanchéité; et
un dispositif d'alimentation pour placer le récipient un par un en dessous de l'aiguille creuse.

4. Dispositif de collecte de substance physiologiquement active selon la revendication 3, comprenant en outre:
un récepteur de résidu liquide (7) positionné entre l'aiguille creuse et le récipient lorsque l'extrémité d'aiguille de l'aiguille creuse se trouve hors du récipient, de manière à recevoir le solvant drainé à travers le trou; et
une second unité d'entraînement configurée de manière à retirer le récepteur de résidu liquide depuis sa position entre l'aiguille creuse et le récipient avant que l'aiguille creuse se déplace vers le bas.

5. Dispositif de collecte de substance physiologiquement active selon la revendication 1,
dans lequel la section de collecte comprend un substrat (12) qui comprend un canal (112) configuré pour l'écoulement du solvant, une entrée de solvant (122) pour le canal, une sortie de solvant (123) pour le canal, et l'ouverture positionnée entre l'entrée et la sortie du canal, et
dans lequel le substrat est remplaçable à partir de la section de collecte.

6. Dispositif de collecte de substance physiologiquement active selon la revendication 5, comprenant en outre:
une section de quantification configurée de manière à quantifier la substance physiologiquement active; et
une section de détermination configurée de manière à déterminer et à acquérir automatiquement des information relatives à l'organisme vivant sur la base de la valeur quantifiée de la substance physiologiquement active.

7. Dispositif de collecte de substance physiologiquement active selon la revendication 6, configuré de telle sorte que des informations relatives au stress soient acquises comme informations relatives à l'organisme vivant, en utilisant des cortisols comme substance physiologiquement active.

8. Dispositif de collecte de substance physiologiquement active selon la revendication 6, configuré de telle sorte que des informations relatives à l'émotion soient acquises comme informations relatives à l'organisme vivant, en utilisant des monoamines comme substance physiologiquement active.

9. Dispositif de collecte de substance physiologiquement active selon la revendication 6, configuré de telle sorte que des informations relatives au cycle menstruel soient acquises comme informations relative à l'organisme vivant, en utilisant des oestrogènes comme substance physiologiquement active.

10. Dispositif de collecte de substance physiologiquement active selon la revendication 6, configuré de telle sorte que des informations relatives à l'effet d'exercice soit acquises comme informations relatives à l'organisme vivant, en utilisant une hormone de croissance comme substance physiologiquement active.
